# EUROPEAN PATENT APPLICATION

(11) **EP 2 322 923 A2**
(43) Date of publication of application: **18.05.2011**
(21) Application number: 11155795.5
(22) Date of filing: 28.06.2006
(51) Int. Cl.: G01N 33/14, G01N 30/88

(54) **Method for procyanidin analysis**

(30) Priority: 30.06.2005 JP 2005191665
(62) Divisional of application: 06780775.0
(71) Applicant: Suntory Holdings Limited, Kita-ku, Osaka-shi Osaka 530-8203 (JP)
(72) Inventor: The designation of the inventor has not yet been filed
(74) Representative: Vossius & Partner

(57) **Abstract**

The present invention relates to a novel method for quantifying procyanidin and flavan-3-ols contained in natural products, foods and drinks, pharmaceuticals and/or cosmetics. The present invention provides a method for quantifying procyanidin (a generic name for a mixture of catechin n-mer; n ≧ 1) contained in test samples such as natural products, foods and drinks, pharmaceuticals and/or cosmetics, which comprises a) pretreating a test sample using a column to remove contaminants which affect the analysis of procyanidin and flavan-3-ol; and b) effecting high performance liquid chromatography (HPLC) to separate and quantify procyanidin and flavan-3-ols in the solution treated to remove contaminants during the above pretreatment step a).

## Description

### FIELD OF THE INVENTION

The present invention relates to a method for quantifying procyanidin and flavan-3-ols contained in natural products, foods and drinks, pharmaceuticals and/or cosmetics.

### BACKGROUND OF THE INVENTION

In terms of efficacy, proanthocyanidin (OPC) is considered to be among the active ingredients related to "French Paradox" because OPC is also contained in wines (1995, Clin. Chim. Acta., 235, 207-219). OPC is known to have various actions and effects such as an antioxidative action, a peripheral circulation-improving action, a blood flow-improving effect and a liver function-improving effect (2004, Japan Food Science, 403, January issue, 40-45), as well as a platelet aggregation-inhibiting effect (JP 2003-527418 A).

Previously-known techniques for proanthocyanidin analysis include reversed-phase HPLC by using a high performance liquid chromatograph-mass spectrometer (LC-MS) (2003, Biosci. Biotechnol. Biochem., 67(5), 1140-1142) and normal phase HPLC by LC-MS using gradient elution (2003, J. Agric. Food Chem., 51, 7513-7521). However, in either of these analysis techniques, procyanidin in the form of a mixture with other ingredients from tea or supplements has been difficult to separate from such contaminant peaks; thus, these techniques cannot achieve accurate quantification. Moreover, there are a very large number of stereoisomers for proanthocyanidin due to the stereoisomerism of its constituents, flavan-3-ols, and compounds available as standards have therefore been limited. For this reason, quantitative analysis has been impossible, except for some known compounds.

### SUMMARY OF THE INVENTION

There is a strong demand for the development of a novel analysis method that enables accurate quantification of procyanidin (a generic name for a mixture of catechin n-mer; n ≧ 1) and flavan-3-ols in tea products, natural products, foods and drinks, pharmaceuticals and/or cosmetics, which contain procyanidin and flavan-3-ols, without receiving any interference from contaminants. Thus, the present invention provides a novel method for quantifying procyanidin and flavan-3-ols contained in tea products, natural products, foods and drinks, pharmaceuticals and/or cosmetics.

### BRIEF DESCRIPTION OF THE DRAWINGS

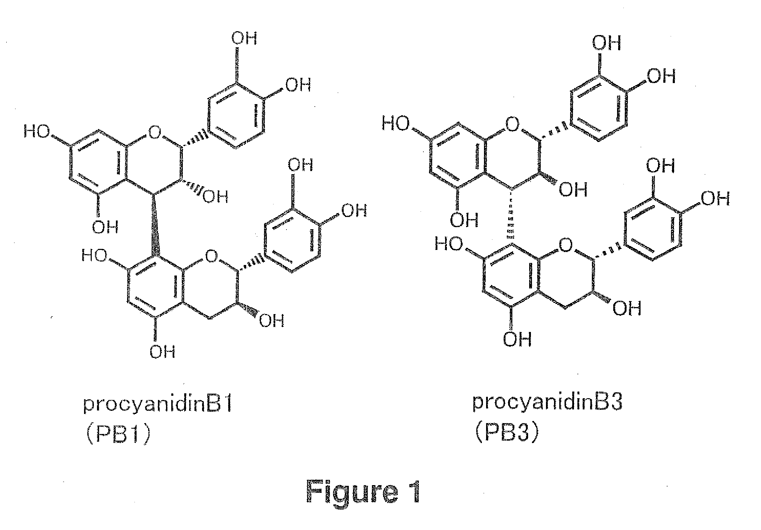

Figure 1 shows the structural formulae of procyanidin B1 and procyanidin B3.

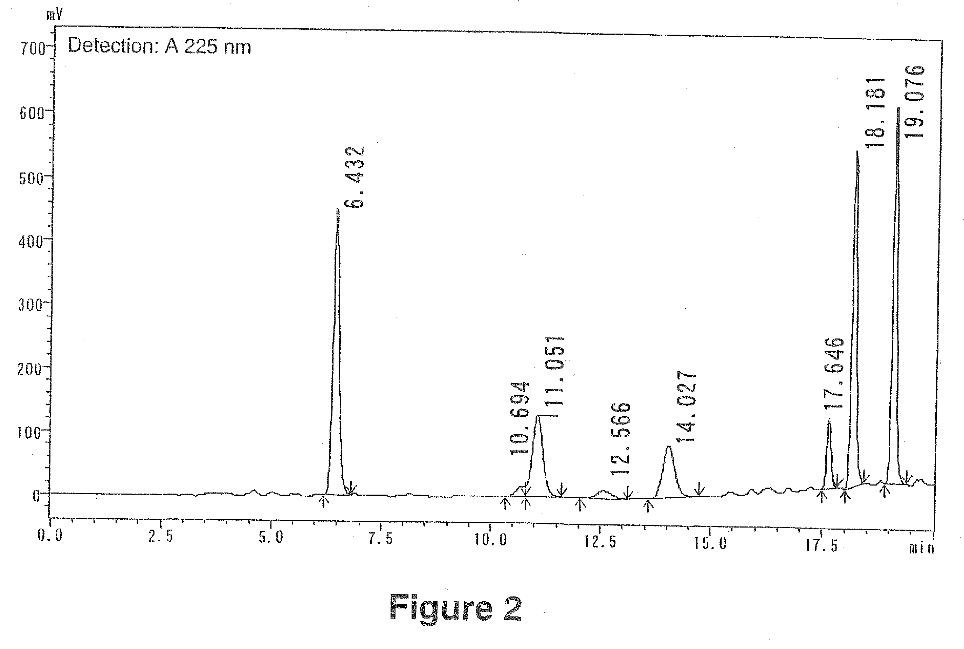

Figure 2 shows an HPLC chromatogram of the fraction eluted with 70% EtOH in the pretreatment step. Analysis conditions are as follows: HPLC system: Shimadzu LC-2010HT (Shimadzu Corporation, Japan); Column: capcellpak C-18 AQ (6 mm x 150 mm, Shiseido Co., Ltd., Japan); Mobile phase: Eluent A: 0.05% TFA/water, Eluent B: 0.05% TFA/90% CH₃CN/water; Flow rate: 1.2 ml/minute; Gradient: 10% B → 10% B (10 minutes) and 10% By → 35% B (10 minutes); Column temperature: 40°C; Detection: A225 nm. Under these conditions, procyanidin B1 was eluted at 10.7 minutes, procyanidin B3 at 12.6 minutes, catechin at 14.0 minutes, epicatechin at 17.6 minutes, gallocatechin at 6..4 minutes, epigallocatechin at 11.1 minutes, epigallocatechin-3-O-gallate at 18.2 minutes, and gallocatechin-3-0-gallate at 19.1 minutes, respectively.

### DETAILED DESCRIPTION OF THE INVENTION

As a result of various studies made to achieve the above object, the inventors of the present invention have found that procyanidin and flavan-3-ols contained in natural products and/or foods and drinks can be quantified without being affected by contaminants when (a) pretreating a test sample with a dextran-based or vinyl polymer-based resin to remove contaminants (e.g., caffeine) which cannot be separated using hydrophobic adsorbent resins or the like, and (b) effecting high performance liquid chromatography to separate and quantify the fractionated procyanidin and flavan-3-ols.

Thus, the present invention provides a method for quantifying procyanidin and flavan-3-ols contained in a test sample, which comprises the following steps:
a) pretreating the test sample using a column to remove contaminants which affect the analysis of procyanidin and flavan-3-ols; and
b) effecting high performance liquid chromatography (HPLC) to separate and quantify procyanidin and flavan-3" ols in the solution treated to remove contaminants during the pretreatment step a).

### Substances to be quantified by the method of the present invention

The substance to be quantified by the method of the present invention may be any of procyanidin (a generic name for a mixture of catechin n-mer; n ≧ 1) and flavan-3-ols. Flavan-3-ols to be quantified by the method of the present invention include, but are not limited to, catechin, epicatechin, gallocatechin, epigallocatechin, catechin-3-O-gallate, epicatechin-3-O-gallate, gallocatechin-3-O-gallate and epigallocatechin-3-O-gallate. Procyanidin to be quantified by the method of the present invention include, but are not limited to, procyanidins B1, B2, B3, B4, B5, B6, B7 and B8. The substance to be quantified by the method of the present invention may preferably be procyanidin B1 (PB1) and/or procyanidin B3 (PB3), and more preferably PB1.

### Test samples

The test sample to be analyzed by the method of the present invention may be any sample expected to contain procyanidin and/or flavan-3-ols, as exemplified by natural products (e.g., grape seeds, tamarind, apple, bark, pine bark-derived polyphenols, tea leaves, cocoa) and/or treated products (e.g., extracts) thereof, foods and drinks, pharmaceuticals, and/or cosmetics. A preferred test sample to be analyzed by the method of the present invention is a tea beverage containing procyanidin, and more preferably a tea beverage containing a pine bark extract.

### Pretreatment step

In the analysis of procyanidin and flavan-3-ols in a test sample using high performance liquid chromatography, contaminants which disturb the analysis include methylxanthines (e.g., caffeine, theobromine), phenylpropanoids (e.g., coffeic acid, coumaric acid, ferulic acid), flavones, flavonols, and glycosylated compounds thereof.

When a reversed-phase (e.g., C8 or C18) carrier is used in an attempt to separate procyanidin and flavan-3-ols from these contaminants, these substances are all adsorbed on the resin and hence eluted at the same time. For this reason, they are impossible to separate from each other or, even where possible, require a very long period of time for their analysis. After a series of studies about conditions which allow separation of procyanidin and flavan-3-ols from these contaminants, the inventors of the present invention have succeeded in separating procyanidin and flavan-3-ols from these contaminants on a gel filtration column with an eluent containing a polar organic solvent. Thus, in the method of the present invention, column separation enables the fractionation of procyanidin and flavan-3-ols from the above contaminants when it is used as a pretreatment.

Column resins available for use in the pretreatment include dextran-based resins (e.g., Sephadex LH-20 (Amersham Biosciences)) and hydrophilic vinyl polymer-based gel filtration resins (e.g., TOYOPEARL HW40 (Tosoh Corporation, Japan), TOYOPEARL HW-50 (Tosoh Corporation, Japan)). These resins enable the fractionation between contaminants and procyanidin/flavan-3-ols. Likewise, a packed column Supardex Peptide 10/300 GL (Amersham Biosciences) can also be used for fractionation in the same manner. However, the column resin is not limited to these examples.

In the pretreatment step, there is no particular limitation on the elution solvent used for charging a test sample onto a column and fractionating procyanidin and flavan-3-ols from contaminants, as long as the solvent achieves fractionation. For this purpose, it is possible to use a mixed solvent of water and a water-soluble polar organic solvent such as ethanol, methanol, acetonitrile or acetone, with ethanol being particularly preferred. For example, after a test sample is charged onto a column, caffeine as a contaminant may be eluted with 0% to 20% ethanol, while phenylpropanoids, flavones and flavonols may be eluted with 20% to 40% ethanol, and finally followed by elution of procyanidin and with 60% to 80% ethanol to obtain the desired procyanidin and flavan-3-ols without containing any contaminant.

### Separation and quantification step by high performance liquid chromatography

Next, the solution treated to remove contaminants in the pretreatment step is analyzed by high performance liquid chromatography (HPLC) to quantify procyanidin and flavan-3-ols contained therein. The column used for the analysis may preferably be a reversed-phase column. Examples include, but are not limited to, reversed-phase columns packed with a packing material of a silica gel carrier having octadecyl groups chemically attached thereto (ODS), a silica gel carrier having butyl groups chemically attached thereto (C4), a silica gel carrier having octyl groups chemically attached thereto (C8), a silica gel carrier having phenyl groups chemically attached thereto (Ph), a silica gel carrier having C30 alkyl chains chemically attached thereto (C30), a synthetic polymer carrier having octadecyl groups attached thereto (ODP), a reversed-phase synthetic polymer carrier (e.g., Shodex Rspak DE series (Showa Denko KK, Japan)), and an amide-functionalized reversed-phase carrier (e.g., Ascentis RIP-Amide (Sigma-Aldrich Japan KK, Japan)).

The mobile phase used for HPLC analysis may be water and a water-soluble polar solvent. It is possible to use ethanol, methanol, acetonitrile, acetone or the like at a mixing ratio of 0% to 100% with water. The mobile phase is preferably acidified for stabilization of ingredients to be separated and analyzed, and may be mixed with an acid such as trifluoroacetic acid (TPA), formic acid, acetic acid, trichloroacetic acid (TCA) or perchloric acid in an amount of 0.001% to 5%, preferably mixed with 0.05% to 0.1% TFA.

More specifically, an ODS column (Capcellpak C-18 AQ (6 mm x 150 mm, Shiseido Co., Ltd., Japan) is used, and gradient elution is performed at a column temperature of 40°C using 0.05% TFA/water (Eluent A) and 0.05% TPA/90% acetonitrile/water (Eluent B) at a flow rate of 1.2 ml/minute with a gradient of 10% B → 10% B (10 minutes) and then 10% B → 35% B (10 minutes). Detection may be accomplished by measuring the absorbance at 225 nm (A225 nm), so that procyanidin and flavan-3-ols can be quantified from the peak areas of these individual substances. Procyanidin B1 (PB1) (Funakoshi Co., Ltd., Japan) and procyanidin B3 (PB3; synthesized according to Example 4 described later) may be used as standards of procyanidin. The structural formulae of PB1 and PB3 are shown in Figure 1. Under the conditions described above, PB1 is eluted at 10.7 minutes and PB3 at 12.6 minutes. Flavan-3-ols, i.e., (-) -epicatechin, (-)-epicatechin 3-O-gallate, (-)-epigallocatechin, (-)-epigallocatechin 3-O-gallate, (+)-catechin, (-)-catechin 3-O-gallate, (+)-gallocatechin and (-)-gallocatechin 3-O-gallate were purchased from Wako Pure Chemical Industries, Ltd., Japan. (-)-Epicatechin was eluted at 17.6 minutes, (-)-epigallocatechin at 11.1 minutes, (-)-epigallocatechin 3-O-gallate at 18.2 minutes, (+)-catechin at 14.0 minutes, (+)-gallocatechin at 6.4 minutes, and (-)-gallocatechin 3-O-gallate at 19.1 minutes. However, analysis conditions are not limited to those described above, and it is possible to use any conditions which allow quantification.

In another embodiment, the method of the present invention may further comprise mass spectrometer analysis after separation by high performance liquid chromatography. Namely, the pretreated sample may also be quantified by LC-MS. Separation and quantification by LC-MS may be accomplished by using, for example, but not limited to, the conditions shown in Example 6 below. In certain cases such as where only procyanidin dimers are to be quantified by LC-MS, such dimers can be quantified from a chromatogram obtained for the ion at m/z 579([M+H]⁺) by selected ion monitoring (SIM). When the amount of procyanidin contained in a sample is very small (1 ppm or less), LC-MS quantification will be an effective analysis means.

The method of the present invention enables the separation of procyanidin and flavan-3-ols from the other contaminants contained in procyanidin-containing tea products, natural products, foods and drinks and/or cosmetics, thereby achieving separation and quantification of procyanidin and flavan-3-ols contained in such tea products and the like. Thus, the method of the present invention is suitable as a method for analyzing procyanidin and flava-3-ols in tea products, natural products, foods and drinks, pharmaceuticals and/or cosmetics, without causing their decomposition.

The present invention will now be described in more detail by way of the following examples, which are not intended to limit the scope of the invention.

### EXAMPLES

### Example 1

### Study of pretreatment conditions

A column containing water-swollen Sephadex LH-20 (dry weight: 0.25 g; Amersham Biosciences) was loaded with 5 ml of an aqueous solution containing proanthocyanidin B1 (PB1) and caffeine (20 ppm each), washed with 2 ml water and then eluted sequentially with 20%, 40%, 60% and 80% EtOH (2 ml each). After concentration under reduced pressure, each eluted fraction was filled up in a 2 ml measuring flask and provided for HPLC (see Example 3 for analysis conditions) to quantify caffeine and PB1.

**Table 1**

| Eluent | % Recovery of caffeine | % Recovery of PB1 |
|---|---|---|
| H₂O (non-adsorbed) | 74% | 0% |
| H₂O (washed) | 23% | 0% |
| 20% EtOH | 2.5% | 0% |
| 40% EtOH | 0% | 0% |
| 60% EtOH | 0% | 49% |
| 80% EtOH | 0.% | 27% |

This result indicated that caffeine was substantially eluted with H₂O, whereas PB1 was eluted with 60% or more EtOH. This means that caffeine and PB1 can be separated from each other.

### Example 2

### Pretreatment of tea product on Sephadex LH-20

A column containing water-swollen Sephadex LH-20 (dry weight: 0.25 g; Amersham Biosciences) was loaded with 5 ml of a tea product containing flavangenol (pine bark-derived procyanidin), washed with 2 ml water and then eluted sequentially with 35% EtOH (2 ml) and 70% EtOH (4 ml). The fraction eluted with 70% EtOH was concentrated under reduced pressure and then filled up in a 2 ml measuring flask. Each eluted fraction was provided for HPLC (see Example 3 for analysis conditions) to quantify caffeine and PB1. The concentration was calculated on the basis of the initial volume (5 ml).

**Table 2**

| Eluent | Caffeine concentration | PB1 concentration |
|---|---|---|
| H₂O (non-adsorbed) | 100 ppm | 0 ppm |
| H₂O (washed) | 9 ppm | 0 ppm |
| 35% EtOH | 0.2 ppm | 0.08 ppm |
| 70% EtOH | 0 ppm | 2.7 ppm |

Caffeine was almost completely eluted by washing with water. Subsequently, phenylpropanoids and flavonols were eluted with 35% EtOH, and the desired procyanidin and flavan-3-ol were eluted with 70% EtOH.

It should be noted that it is also possible to use a tea product which has been taken in a volume of 5 ml, freeze-dried and then reconstituted with 5 ml water before use.

### Example 3

### HPLC separation of procyanidin and flavan-3-ols

The fraction treated on Sephadex LH-20 and eluted with water, 35% EtOH or 70% EtOH, as well as the sample solution pretreated to remove contaminants such as caffeine were analyzed by HPLC under the following conditions.

Analysis conditions: Capcellpak C-18 AQ (6 mm x 150 mm, Shiseido Co., Ltd., Japan) was used as a column, and gradient elution was performed using 0.05% TFA/water (Eluent A) and 0.05% TFA/90% CH₃CN/water (Eluent B) at a flow rate of 1.2 ml/minute with a gradient of 10% B → 10% B (10 minutes) and then 10% B → 35% B (10 minutes). The column temperature was set to 40°C, and detection and quantification were based on measurements of A225 nm and its peak area. The HPLC used was Shimadzu LC-2010HT (Shimadzu Corporation, Japan).

Under these conditions, procyanidin B1 was eluted at 10.7 minutes, procyanidin B3 at 12.6 minutes, catechin at 14.0 minutes, epicatechin at 17.6 minutes, gallocatechin at 6.4 minutes, epigallocatechin at 11.1 minutes, epigallocatechin-3-O-gallate at 18.2 minutes, and gallocatechin-3-O-gallate at 19.1 minutes. This result showed good separation of these substances (Figure 2).

### Example 4

### Synthesis of procyanidin B3

Procyanidin B3 was synthesized as follows, according to the article (J. C. S. Perkin I, 1981, Jacobus J. Botha, et al., 1235-1245).

(+)-Taxifolin (500 mg) was dissolved in ethanol (50 ml), followed by addition of NaBH₄ (200 mg). After (+)-catechin (1 g) was added and dissolved, the resulting mixture was mixed with HCl and stirred for 1 hour. The reaction product was purified by reversed-phase HPLC to give procyanidin B3 (200 mg).

### Example 5

### Study of resins for use in pretreatment

A column containing water-equilibrated TOYOPEARL HW-40 (1 ml, Tosoh Corporation, Japan) was loaded with 2 ml of an aqueous solution containing procyanidin B1 (PB1) and caffeine (20 ppm each), washed with 2 ml water and then eluted sequentially with 35% EtOH (2 ml) and 70% EtOH (4 ml). Each eluted fraction was provided for HPLC to quantify caffeine and PB1.

**Table 3**

| Eluent | % Recovery of caffeine | % Recovery of PB1 |
|---|---|---|
| H₂O (Non-adsorbed and washed) | 97.3% | 0% |
| 35% EtOH | 2.5% | 1.3% |
| 70% EtOH | 0% | 77% |

This result indicated that caffeine was substantially eluted with H₂O whereas PB1 was eluted with 70% EtOH, as in the case of Sephadex LH-20. This means that caffeine and PB1 can be separated from each other. This pretreatment method was therefore regarded as an effective method with dextran-based gel filtration resins or hydrophilic vinyl polymer-based resins.

### Example 6

### Quantification with mass spectrometer

The pretreated sample was quantified by LC-MS under the following conditions.

The mass spectrometer used was Quattro micro (Micromass, Manchester, UK) and the measurement was performed in ESI positive mode under the following conditions:

| | |
|---|---|
| Cone voltage | 35 V |
| Collision energy | 2 eV |
| Capillary voltage | 4000 V |
| Source block Temp. | 80°C |
| Desolvation Temp. | 350°C. |

The HPLC used was Aliance 2795 (Nihon Waters KK, Japan). HPLC conditions are as shown below.
Column: Capcell park C-18 AQ (3 mm φ x 15 cm, Shiseido Co., Ltd., Japan)
Mobile phase: Eluent A: 0.1% HCOOH/water, Eluent B: 90% CH₃CN/0.1% HCOOH/water, flow rate 0.2 ml/minute
Gradient: 10% B → 35% B (7 minutes) → 70% B (3 minutes)

Under these conditions, PB1 was eluted at 7.3 minutes and PB3 at 7.8 minutes. Quantification was performed using a calibration curve prepared for the chromatographic area at m/z 579([M+H]') in selected ion monitoring (SIM). When the amount of procyanidin contained in a sample is very small (1 ppm or less), LC-MS quantification will be an effective analysis means.

## Claims

1. A method for quantifying procyanidin (a generic name for a mixture of catechin n-mer: n ≧ 1) and flavan-3-ols contained in a test sample, which comprises the following steps:
a) pretreating the test sample using a column to remove contaminants which affect the analysis of procyanidin and flavan-3-ols; and
b) effecting high performance liquid chromatography (HPLC) to separate and quantify procyanidin and flavan-3-ols in the solution treated to remove contaminants during the pretreatment step a).

2. The method according to claim 1, wherein the contaminants are one or more substances selected from the group consisting of caffeine, theobromine, phenylpropanoids, flavonols, and glycosylated compounds of phenylpropanoids and flavonols.

3. The method according to claim 1 or 2, wherein the test sample is a food or a drink.

4. The method according to any one of claims 1 to 3, wherein the test sample is a tea beverage containing procyanidin.

5. The method according to any one of claims 1 to 3, wherein the test sample is a tea beverage containing a pine bark extract.

6. The method according to any one of claims 1 to 5, wherein the pretreatment step comprises using a column of a dextran-based or vinyl polymer-based resin.

7. The method according to any one of claims 1 to 6, wherein the pretreatment step is carried out under conditions where a water/ethanol mixture is used, thereby separating procyanidin and flavan-3-ols from contaminants.

8. The method according to any one of claims 1 to 7, wherein the pretreatment step comprises using a column of a dextran-based resin, equilibrating the column with water, loading the sample onto the column, eluting contaminants with water and/or 0% to 40% ethanol, and then collecting procyanidin and flavan-3-ols with 60% or more ethanol.

9. The method according to any one of claims 1 to 6, wherein the pretreatment step is carried out under conditions where a mixed solution selected from the group consisting of a water/methanol mixture, a water/ethanol mixture, a water/acetonitrile mixture and a water/acetone mixture is used, thereby separating procyanidin and flavan-3-ols from contaminants.

10. The method according to any one of claims 1 to 9, wherein the column for high performance liquid chromatography used in step b) is a reversed-phase column.

11. The method according to claim 10, wherein the reversed-phase column is a column packed with a packing material selected from the group consisting of a silica gel carrier having octadecyl groups chemically attached thereto (ODS), a silica gel carrier having butyl groups chemically attached thereto (C4), a silica gel carrier having octyl groups chemically attached thereto (C8), a silica gel carrier having phenyl groups chemically attached thereto (Ph), a silica gel carrier having C30 alkyl chains chemically attached thereto (C30), a synthetic polymer carrier having octadecyl groups attached thereto (ODP), a reversed-phase synthetic polymer carrier, and an amide-functionalized reversed-phase carrier.

12. The method according to any one of claims 1 to 11, wherein the mobile phase used in step b) for separation by high performance liquid chromatography is acetonitrile and/or water, each of which may or may not contain TFA (trifluoroacetic acid).

13. The method according to claim 12, wherein the mobile phase is a gradient of acetonitrile and water, each of which may or may not contain TFA (trifluoroacetic acid).

14. The method according to any one of claims 1 to 11, wherein the mobile phase used in step b) for separation by high performance liquid chromatography comprises a solvent selected from the group consisting of methanol, ethanol, acetonitrile, acetone and a mixed solvent thereof.

15. The method according to claim 14, wherein the mobile phase used in step b) for separation by high performance liquid chromatography further comprises TFA (trifluoroacetic acid), formic acid, acetic acid, TCA (trichloroacetic acid) or perchloric acid.

16. The method according to any one of claims 1 to 15, wherein the method is for quantifying procyanidin and the procyanidin to be quantified is procyanidin B1 (PB1) and/or procyanidin B3 (PB3).

17. The method according to any one of claims 1 to 15, wherein the procyanidin to be quantified is procyanidin B1 (PB1).

18. The method according to any one of claims 1 to 17, which is further combined with a mass spectrometer in order to quantify only procyanidin dimers.
